# EUROPEAN PATENT SPECIFICATION

(11) **EP 1 363 534 B1**
(45) Date of publication and mention of the grant of the patent: **28.05.2008**
(21) Application number: 02732201.5
(22) Date of filing: 10.01.2002
(51) Int. Cl.: A61B 5/05, A61B 5/053

(54) **DEVICE FOR NON-DESTRUCTIVE AND NON-INVASIVE CLINICAL IMPEDANCE MEASUREMENTS OF SKIN**
VORRICHTUNG FÜR NICHT-DESTRUKTIVE UND NICHT-INVASIVE KLINISCHE IMPEDANZMESSUNGEN AN DER HAUT
DISPOSITIF DESTINE A DES MESURES D'IMPEDANCE CLINIQUES NON DESTRUCTIVES ET NON INVASIVES DE LA PEAU

(30) Priority: 22.01.2001 SE 0100168
(43) Date of publication of application: 26.11.2003
(73) Proprietor: Sethson, Britta, 905 93 Umea (SE); Geladi, Paul, 906 54 Umea (SE)
(72) Inventor: Sethson, Britta, 905 93 Umea (SE); Geladi, Paul, 906 54 Umea (SE)
(74) Representative: Onn, Thorsten
(86) International application number: PCT/SE2002/000031
(87) International publication number: WO 2002/056766

(56) References cited:
- WO-A-00/62671
- US-A- 4 375 219
- US-A- 5 588 440
- US-A- 5 897 505
- Kungliga teknisha hogskolan och Karolinska institutet. institutionen for medicinsk laboratorievetenskap och teknik. Enheten for medicinsk teknik. Stockholm Ht 1996, rapport 22, P. Blidefalk et al.: matning av elektrisk impedans icke-invasivt in vivo, samt analys och tolkning av data, pages 1-42

## Description

The present invention relates to a device for non-destructive and non-invasive clinical measurements of skin according to the introduction to the enclosed independent claim.

There is a clear trend towards non-destructive and non-invasive clinical measurements. Different sensors based on electrochemistry, spectroscopy, etc. are already used today to measure in or through the skin of different parts of the body. These are here designated *"spectral sensors".* The spectral sensors are usually built into a holder that together is designated a *"probe".*

It is known that the local measurement environment has a significant influence on the measurement result from such spectral sensors. Factors that influence the result are, among others, humidity and temperature, to both of which almost all spectral sensors are sensitive. In addition, both the application pressure and the angle of contact between the skin and the sensor must be correct and reproducible when the spectral sensor touches the skin. The angle of contact must be 0 degrees. Taken as a whole, this is a significant problem since nursing staff with little training in handing probes must be able to use them in the correct manner.

Most commercial probes have a diameter in the range 3 to 20 mm and are open. Both of these factors make it hard to attain a constant volume of skin at the moment of measurement where humidity and temperature equilibrium can be reached. The necessary establishment of humidity and temperature equilibrium becomes more difficult the larger the probe is since it consumes or gives off heat energy to the skin. Furthermore, as the skin is not flat over large surfaces, it is difficult to obtain the correct angle of contact and reproducible application pressure. It is well known that the measurement is influenced by the size of the probe but not always how large the influence that occurs is, or how quickly equilibrium can be established.

A known apparatus according to the description above and to the first part of claim 1, made for measuring relative hydration of a substrate, is shown and described in WO 00/62671, A1. This apparatus has a temperature sensor and first and second spaced electrodes. All the sensors or sensor parts are placed on line on the tip of the apparatus, on the same level. When the tip is placed against the substrate the whole face of the tip, and all sensors, will be placed directly against the substrate.

When measuring the impedance of the skin, we have used a commercial rod-shaped measuring probe (here called impedance rod) where the flat tip constitutes the actual U.; Geladi, P. Multivariate analysis of skin impedance data in long term type 1 diabetic patients. Chemometrics and intelligent laboratory systems, 1998,44, 381), it would be valuable to be able to simplify the measuring procedure so that more reproducible values can be obtained for more reliable diagnoses.

It is the aim of the resent invention to reduce or overcome the drawbacks that are found within known technology.

This aim is achieved with a device first named above and that has the features that are defined in the characteristics section of claim 1.

Other features and benefits of the invention and its embodiments will be evident from the dependent claims and from the following detailed description of embodiments of the invention.

The invention means more knowledge of how the measurement is performed and can prevent erroneous measurements being made.

Miniaturised temperature, pressure and humidity sensors can be used in combination with the spectral sensor. Patient and time-based integration of the measurement data with the spectral data is desirable and can be possible with this combination.

Two published articles have shown that information about early alterations in the skin of diabetics can be deciphered by such skin impedance measurements *(*Lindholm-Sethson, B.; Han, S., Ollmar, S.; Nicander, I.; Jonsson, G.; Lithner, F.; Bertheim, U.; Geladi, P. Multivariate analysis of skin impedance data in long term type 1 diabetic patients. Chemometrics and intelligent laboratory systems, 1998, 44, 381*)* and by NIR measurements *(*Geladi, P.; Nyström, J.; Eriksson, J.W.; Nilsson, A.; Lithner, F.; Lindholm-Sethson, B. A multivariate NIR study of skin alterations in diabetic patients as compared to control subjects, Journal of Near Infrared Spectroscopy, 8.5, 2000 in press*).*

In one embodiment of the present invention, we are able to make the measurements reproducible and stable by combining both these methods of measurement in the same measurement probe.

In addition, by designing the carrier of the complementary sensors so that it can be arranged on an existing probe, e.g. the previously named impedance rod, a flexible and cost-effective device is achieved.

In the following detailed description of embodiments of the invention, special expressions and terms for the component parts have been chosen to clarify the embodiments. As such, these expressions and terms should not be interpreted as limitations of the invention, but rather as examples within its scope of protection.

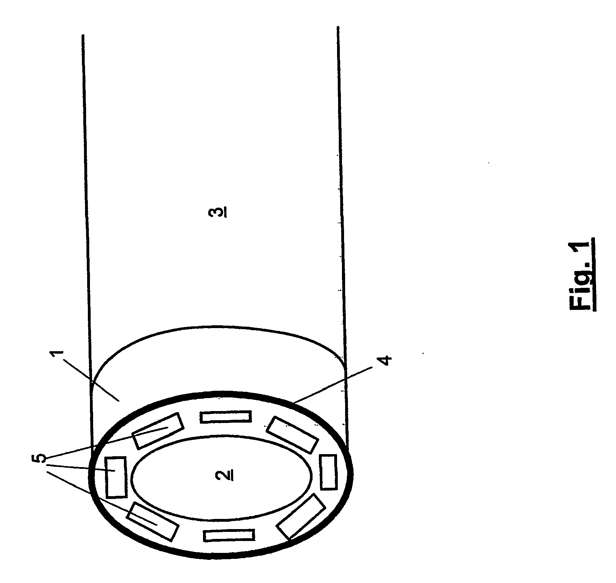

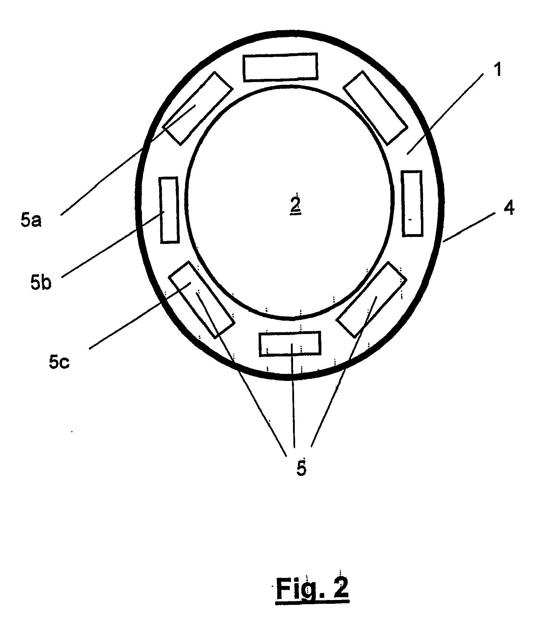

Fig. 1 shows schematically a perspective view of the active contact surface of a measuring tool according to a first embodiment of the present invention and Fig. 2 shows a plane view of the arrangement according to Fig. 1.

The arrangement includes a holder having a flat spectral sensor 2 at an active end. Complementary sensors 5, preferably miniature sensors, for reading the measurement environment of the spectral sensor are arranged surrounding the spectral sensor 2 and in essentially the same plane.

A means of demarcation 4 is arranged to extend around the complementary sensors 5, whereby the means of demarcation 4 is, together with the holder 1, 3, able to demarcate a measuring area that includes all the sensors 2, 5 when the device is brought against the skin. The means of demarcation 4 can be a so-called O-ring or similar level edge-shaped and continuous sealing.

The complementary sensors 5 for measuring the local measurement environment include temperature sensors 5a, pressure sensors 5b and humidity sensors 5c.

The complementary sensors 5 are supported by a ring-shaped holder 1 having an internal, complementarily-shaped hole for its removable arrangement over the active end of a commercially available probe having only a spectral sensor, whereby all the probes 2, 5 are arranged in the same plane in the assembled position of the ring.

One embodiment of the present invention is described with reference to Fig. 1. It includes a concentric ring 1 with outer and inner diameters. Ring 1 is arranged around the active end 2 of a probe 3 that has an active plane and circularly-executed spectral sensor 2.

The ring 1 has a peripheral O-ring 4 of polymer material that demarcates a measuring area and that makes it possible to maintain temperature and humidity in the measuring area.

Ring 1 has places for up to eight miniature sensors 5 arranged essentially in the same plane as the spectral sensor 2. These miniature sensors measure temperature and humidity as well as pressure and, via differential measurement, geometry.

Fig. 2 shows how the ring 1 in the embodiment according to Fig. 1 is arranged around a spectral sensor 2.

In another embodiment, the invention is an addition to an existing probe. It can be attached to most probes through adapting the inner diameter of the ring to the outer diameter of the probe 3, usually 3-20 mm. The ring 1 creates the prerequisites for a constant volume at the measuring occasion where humidity and temperature equilibrium can be attained after a time through mutual heat exchange between the probe and the skin.

In one preferred embodiment, the ring 1 has one to three temperature sensors and one to two humidity sensors in miniature format. In addition, it can have two to four miniature pressure sensors that monitor the application pressure of the probe against the skin and how the pressure is distributed.

All sensors give digital results that can be collected in a file of data on a data storage medium. If spectra are measured, a combined file of data with spectrum-sensor data is obtained.

The are two main methods for using the device according to the invention; firstly, temperature, humidity, pressure and pressure distribution are measured during measurement with the spectral sensor and secondly, measurement with the spectral sensor is activated when temperature, humidity, pressure and geometry are measured to be within a specified interval, i.e. when the specified local measurement environment is established.

## Claims

1. Device for non-destructive and non-invasive clinical measurements of skin including a holder (1, 3) having a flat spectral sensor (2) at its active end, and complementary sensors (5) arranged around the spectral sensor (2) essentially in the same plane for reading the measurement environment of the spectral sensor, **characterised in that** the spectral sensor (2) is adapted to carry out NIR and impedance-measurements, and that the complementary sensors (5) are arranged in a removable manner on a probe that includes the spectral sensor (2).

2. Device according to claim 1, **characterised in that** a means of demarcation (4) is arranged to extend around the complementary sensors (5), whereby the means of demarcation (4) is, together with the holder (1, 3), able to demarcate a measuring area that includes all the sensors (2,5) when the device is brought against the skin.

3. Device according to claim 1 or 2, **characterised in that** the complementary sensors (5) for reading the local measurement environment include temperature sensors (5a).

4. Device according to claims 1 - 3, **characterised in that** the complementary sensors (5) for reading the local measurement environment include pressure sensors (5b).

5. Device according to claims 1 - 4, **characterised in that** the complementary sensors (5) for reading the local measurement environment include humidity sensors (5c).

6. Device according to claim 1, **characterised in that** the complementary sensors (5) are supported by a ring-shaped holder (1) having an internal, complementarily-shaped hole for the removable arrangement over the active end of a commercially available probe having only a spectral sensor, whereby all the probes (2, 5) are arranged in the same plane in the assembled position of the ring.

## Patentansprüche

1. Vorrichtung für nicht-destruktive und nicht-invasive klinische Hautmessungen mit einer Halterung (1, 3), die an ihrem aktiven Ende einen flachen Spektralsensor (2) aufweist sowie Komplementärsensoren (5), die im wesentlichen in der gleichen Ebene um den Spektralsensor (2) angeordnet sind, um die Messumgebung des Spektralsensors zu erfassen, **dadurch gekennzeichnet, dass** der Spektralsensor (2) zur Durchführung von NIR- und Impedanzmessungen ausgebildet ist, und dass die Komplementärsensoren (5) abnehmbar an einer Sonde angebracht sind, die den Spektralsensor (2) umfasst.

2. Vorrichtung nach Anspruch 1, **dadurch gekennzeichnet, dass** um die Komplementärsensoren (5) ein Abgrenzungsmittel (4) angeordnet ist, wobei das Abgrenzungsmittel (4) zusammen mit der Halterung (1, 3) in der Lage ist, einen Messbereich zu begrenzen, der alle Sensoren (2, 5) umfasst, wenn die Vorrichtung gegen die Haut gehalten wird.

3. Vorrichtung nach Anspruch 1 oder 2, **dadurch gekennzeichnet, dass** die Komplementärsensoren (5) zur Erfassung der lokalen Messumgebung Temperatursensoren (5a) beinhalten.

4. Vorrichtung nach den Ansprüchen 1 bis 3, **dadurch gekennzeichnet, dass** die Komplementärsensoren (5) zur Erfassung der lokalen Messumgebung Drucksensoren (5b) beinhalten.

5. Vorrichtung nach den Ansprüchen 1 bis 4, **dadurch gekennzeichnet, dass** die Komplementärsensoren (5) zur Erfassung der lokalen Messumgebung Feuchtigkeitssensoren (5c) beinhalten.

6. Vorrichtung nach Anspruch 1, **dadurch gekennzeichnet, dass** die Komplementärsensoren (5) an einer ringförmigen Halterung (1) angebracht sind, die innen ein komplementär geformtes Loch zum abnehmbaren Aufstecken auf das aktive Ende einer handelsüblichen Sonde mit nur einem Spektralsensor aufweist, wobei alle Sonden (2, 5) in der zusammengefügten Stellung des Rings in der gleichen Ebene liegen.

## Revendications

1. Dispositif destiné à des mesures cliniques non destructives et non invasives de la peau, comprenant un support (1, 3) ayant un capteur spectral (2) plat à son extrémité active et des capteurs complémentaires (5) agencés autour du capteur spectral (2) essentiellement dans le même plan pour détecter l'environnement de mesure du capteur spectral, **caractérisé en ce que** le capteur spectral (2) est adapté à effectuer des mesures dans le proche infrarouge et d'impédance, et que les capteurs complémentaires (5) sont agencés de manière amovible sur une sonde qui comprend le capteur spectral (2).

2. Dispositif selon la revendication 1, **caractérisé en ce qu'**un moyen de démarcation (4) est agencé autour des capteurs complémentaires (5), le moyen de démarcation (4) étant capable de démarquer avec le support (1, 3) une zone de mesure qui comprend tous les capteurs (2, 5) lorsque le dispositif est amené contre la peau.

3. Dispositif selon la revendication 1 ou 2, **caractérisé en ce que** les capteurs complémentaires (5) pour détecter l'environnement de mesure local comprennent des capteurs de température (5a).

4. Dispositif selon les revendications 1 à 3, **caractérisé en ce que** les capteurs complémentaires (5) pour détecter l'environnement de mesure local comprennent des capteurs de pression (5b).

5. Dispositif selon les revendications 1 à 4, **caractérisé en ce que** les capteurs complémentaires (5) pour détecter l'environnement de mesure local comprennent des capteurs d'humidité (5c).

6. Dispositif selon la revendication 1, **caractérisé en ce que** les capteurs complémentaires (5) sont supportés par un support (1) annulaire ayant un trou intérieur de forme complémentaire pour sa fixation amovible sur l'extrémité active d'une sonde commerciale ayant un capteur spectral uniquement, toutes les sondes (2, 5) étant situées dans le même plan en position assemblée de l'anneau.
